# EUROPEAN PATENT APPLICATION

(11) **EP 3 409 199 A1**
(43) Date of publication of application: **05.12.2018**
(21) Application number: 17744178.9
(22) Date of filing: 24.01.2017
(51) Int. Cl.: A61B 5/11, A61C 19/04

(54) **ARTIFICIAL FOOD BOLUS FOR MASTICATORY FUNCTION TRAINING, ARTIFICIAL BOLUS FOR MASTICATORY FUNCTION EVALUATION, AND SYSTEM FOR MANAGING EVALUATION OF MASTICATORY FUNCTION USING SAME**

(30) Priority: 25.01.2016 JP 2016011462; 29.03.2016 JP 2016065696
(71) Applicant: Examastica Co., Tokyo 168-0064 (JP)
(72) Inventor: NAKAMURA, Toru, Oyama-shi Tochigi 329-0206 (JP); HAYAFUNE, Koji, Tokyo 168-0064 (JP); AMAYA, Shinji, Saitama-shi Saitama 336-0017 (JP); HIGUCHI, Akira, Tokyo 153-0043 (JP); SATOU, Nobuo, Matsudo-shi Chiba 271-0075 (JP)
(74) Representative: Wills, Andrew Jonathan
(86) International application number: PCT/JP2017/002307
(87) International publication number: WO 2017/130948

(57) **Abstract**

[Problem] To provide a masticatory-function evaluation and management system capable of quickly counting "fine particles having been not crushed and ground finely" and quickly quantifying masticatory efficiency using photographic data of an artificial food mass in a stretched state, and capable of managing the masticatory efficiency quantified using such an artificial food mass for masticatory function evaluation along the time series. [Solution] When image data of an artificial food mass, in a stretched state, for masticatory function evaluation is received, the image data being transmitted from a user terminal, the number of fine particles having a spherical shape is counted from among all fine particles present in the image data, and masticatory efficiency is calculated on the basis of the number of the fine particles having the spherical shape.

## Description

### Technical Field

The present invention relates to an artificial food mass for masticatory function training, which is adapted for training of mastication with the aid of the artificial food mass containing fine particles, an artificial food mass for masticatory function evaluation, which enables the human masticatory function to be quickly and easily evaluated with the aid of the artificial food mass containing the fine particles, and a masticatory-function evaluation and management system using the latter artificial food mass.

### Background Art

Mastication, namely actions of chewing foods, sufficiently mixing the foods with saliva, and forming a wet food mass having a suitable size to be ready for swallowing the food mass, is regarded as necessary for a human being to eat solid foods, and as beneficial to provide not only the awakening effect and the relaxing effect, but also the other effects of preventing adiposity, dementia, visual degradation, worsening of posture, tooth decay, and cancer, and increasing the volume of blood in the brain, thereby suppressing aging changes.

The ability of the mastication (i.e., masticatory efficiency) is affected by many factors, such as the number of teeth, soundness of the teeth, forms of the occlusal surfaces, states of the periodontal tissues, jawbone forms, strength of the masticatory muscles, lower-jaw motion modes, age, and states of the prostheses.

Aiming to quantify the masticatory efficiency, the inventors of this application have developed an artificial food mass containing "substantially-uniform spherical fine particles having properties that the particles are crushed and ground finely by mastication", and a system of evaluating the masticatory function with the aid of the artificial food mass (Patent Literature (PTL) 1).

The system of evaluating the masticatory function includes the steps of letting a person masticate the artificial food mass containing the fine particles that maintain a spherical shape when they are not crushed and ground finely by the mastication, placing the masticated artificial food mass between two prepared slides and stretching the artificial food mass up to an appropriate thickness to such an extent as not squashing the fine particles, and counting the number of fine particles that remain in the stretched artificial food mass between the two prepared slides and still have the spherical shape.

The artificial food mass for use in the system of evaluating the masticatory function is formed, for example, by preparing Carnauba wax, which is one type of natural wax, into substantially-uniform spherical fine particles, mixing those fine particles into a base material made of, e.g., polyisobutylene, and kneading the mixture.

It is generally known that, during mastication, upper and lower teeth are each displaced about 100 µm. Taking such a point into consideration, outer diameters of the fine particles are set to about 100 to 500 µm to ensure that the "fine particles are crushed and ground finely by the mastication".

The masticatory efficiency is quantified by letting the person masticate the artificial food mass containing the fine particles that have the above-mentioned sizes, placing the masticated artificial food mass between two prepared slides, stretching the artificial food mass up to an appropriate thickness to such an extent as not squashing the fine particles, and counting the number of fine particles that remain in the stretched artificial food mass between the two prepared slides and still have the spherical shape.

In order to inspect the masticatory efficiency with high accuracy, about 2000 fine particles are contained in the artificial food mass, and those fine particles are uniformly dispersed in the artificial food mass. Therefore, an error of the inspection result can be suppressed which may occur due to individual differences among the many artificial food masses (such as differences in the number of the fine particles contained in the individual artificial food masses).

The number of the "fine particles not crushed and ground finely" is visually counted by an evaluator, or counted in a manner of taking an image of the food mass by a CCD (Charge Coupled Device) camera, and performing, e.g., pattern matching by a computer, for example.

In connection with the case of performing, e.g., the pattern matching by a computer, for example, it is also proposed, for the purpose of clarifying a difference in color between the artificial food mass and the fine particles, to employ the fine particles having optical transparency, to irradiate the artificial food mass with lights in difference wavelength bands from above and below at the time of taking the image of the artificial food mass, and to obtain the image in which the color of the fine particles having optical transparence and the color of the base material having low optical transmittance are apparently different from each other (PTL 2).

### Citation List

### Patent Literatures

PTL 1: International Publication No. WO2008/020588
PTL 2: JPA 2011-103586

### Summary of Invention

### Technical Problem

However, when the fine particles of about 100 to 500 µm are counted by performing, e.g., the pattern matching, a dedicated counting system constituted by an imaging device, a computer, and so on is required, and the masticatory function cannot be easily evaluated.

In some cases, an evaluator visually counts the fine particles for the purpose of evaluating the masticatory function in a simplified manner using the artificial food mass that contains the fine particles. However, it is difficult to visually count the fine particles of about 100 to 500 µm with accuracy. Hence a lot of time and skill are required for the counting.

Thus, a difficulty resides in quickly quantifying the masticatory efficiency in the field of dental care, and in easily quantifying the masticatory efficiency of individual persons by themselves. In most cases, therefore, the artificial food mass in the state sandwiched between the two prepared slides, for example, is delivered to and inspected by, e.g., a business facility equipped with the dedicated counting system. This means that a time necessary for the delivery is taken.

Moreover, when the masticatory function is determined to be low as a result of evaluating the masticatory function as described above, a dentist, for example, tries to guide training of the mastication in some cases.

In general, although guidance in points of "chewing slowly and properly", "chewing foods about 30 times before swallowing", etc. can be given, putting such guidance into practice is difficult to continue in a conscious way.

For that reason, commodities using a gum base slightly harder than that of a usual chewing gum and being expected to increase the masticatory function are also commercialized as represented, for example, by DAY-UP "Oral Gum <Chewing Training>" made by Lion Corporation.

The chewing gum using the relatively hard gum base is expected to provide a certain effect in promoting growth and development of jawbones and the strength of the masticatory muscles, but urging a user to continue the "conscious mastication" is difficult in practice.

In addition, a training gum using the relatively hard gum base accompanies with a risk that not only the teeth, but also the periodontal tissues such as the periodontal membrane may be damaged when a user excessively chews the gum with full force or continuously chews the gum for a long time.

In consideration of the above-described situation, an object of the present invention is to provide an artificial food mass for masticatory function evaluation, which enables the masticatory efficiency to be quickly quantified by quickly counting "fine particles having been not crushed and ground finely" with reference to photographic data of an artificial food mass in a stretched state,, the photographic data being taken by a smartphone, for example.

Another object of the present invention is to provide a masticatory-function evaluation and management system capable of managing the masticatory efficiency quantified using the above artificial food mass for masticatory function evaluation along the time series.

Still another object of the present invention is to provide an artificial food mass for masticatory function training, with which a user can perform training to practice the "conscious mastication" and to increase the masticatory efficiency.

### Solution to Problem

The present invention has been accomplished with intent to solve the above-described problems in the related art, and provides an artificial food mass for masticatory function evaluation used to evaluate human masticatory function,
the artificial food mass including a base material and fine particles contained in the base material,
wherein the fine particles have a spherical shape when not chewed to be crushed and ground finely with mastication, and
outer diameters of the fine particles are 500 µm to 2 mm.

In the above artificial food mass for masticatory function evaluation, preferably, a number 10 to 500 of the fine particles are contained per 1 g of the artificial food mass for masticatory function evaluation.

The fine particles may be formed of edible capsules, wax, sugar, or plant seeds.

The present invention further provides a masticatory-function evaluation and management system including an analyzer and performing evaluation and management of human masticatory function,
wherein the analyzer counts the number of fine particles having a spherical shape from among the fine particles present in image data of one, in a stretched state, of the artificial food masses for masticatory function evaluation described above, and calculates masticatory efficiency on the basis of the number of the fine particles having the spherical shape.

The analyzer may be a user terminal.

In that case, the masticatory-function evaluation and management system may further include a server computer connected to the user terminal via a network,
the server computer may include a masticatory-function evaluation and management database and database management means to input and output data into and from the masticatory-function evaluation and management database, and
the masticatory-function evaluation and management database may be constituted such that the masticatory efficiency is registerable in link with examinee information.

As an alternative, the analyzer may be a server computer connected to a user terminal via a network, and
upon receiving image data of one, in a stretched state, of the artificial food masses for masticatory function evaluation described above, the image data being sent from the user terminal, the server computer may count the number of fine particles having a spherical shape from among the fine particles present in the image data, and may calculate masticatory efficiency on the basis of the number of the fine particles having the spherical shape.

In the above case, the server computer may include a masticatory-function evaluation and management database and database management means to input and output data into and from the masticatory-function evaluation and management database, and
the masticatory-function evaluation and management database may be constituted such that the masticatory efficiency is registerable in link with examinee information.

The present invention still further provides an artificial food mass for masticatory function training used to practice training of mastication,
the artificial food mass including a base material and fine particles contained in the base material,
wherein outer diameters of the fine particles are 500 µm or more, and
compression strength of the fine particles is greater than compression strength of the base material.

In the above artificial food mass for masticatory function training, preferably, the compression strength of the fine particles is 4.0 kgw/mm² or less.

The fine particles may be formed of edible capsules, wax, sugar, or plant seeds.

### Advantageous Effects of Invention

According to the present invention, since the outer diameters of the fine particles are set to 500 µm to 2 mm, the fine particles can be easily observed even in a visual way, and the human masticatory function can be quickly and easily evaluated by an examinee himself or herself.

According to the present invention, since the evaluated masticatory function is managed along the time series, changes in the masticatory function can be clearly grasped, and the masticatory function can be grasped and managed by the examinee himself or herself.

Therefore, an enlightening effect of increasing the consciousness of the mastication can be given to the examinee. Another advantageous effect is that, for instance, when the masticatory function is reduced, the examinee may be prompted to receive diagnosis by a dentist.

Furthermore, also in the field of dental care, the masticatory function can be quickly and easily evaluated on site. Hence the dentist can easily give education and enlightenment regarding the mastication to the examinee.

In addition, according to the present invention, in training intended to increase the masticatory function, a user can perceive a feeling of crushing the fine particles due to the difference in compression strength between the base material and the fine particles. Thus, since the user can consciously crush the fine particles during the mastication, it is possible to urge the user to practice the "conscious mastication", and to perform the training to increase the masticatory function.

As a result, the necessity of making harder the base material (gum base) of the artificial food mass is no longer present, and the user is kept from excessively chewing the artificial food mass with full force. Moreover, even if the user continues to chew the artificial food mass for a long time, the teeth, the periodontal tissues, etc. can be avoided from being damaged.

### Brief Description of Drawings

[Fig. 1] Fig. 1 is a schematic view referenced to explain an artificial food mass for masticatory function evaluation according to the present invention.
[Fig. 2] Fig. 2 is a schematic view referenced to explain a state of the artificial food mass for masticatory function evaluation, illustrated in Fig. 1, after stretching the artificial food mass.
[Fig. 3] Fig. 3 is a block diagram referenced to explain an example of a masticatory-function evaluation and management system according to the present invention.
[Fig. 4] Fig. 4 is an illustration referenced to explain a structure of a masticatory-function evaluation and management database used in the masticatory-function evaluation and management system illustrated in Fig. 3.
[Fig. 5] Fig. 5 is a schematic view referenced to explain an artificial food mass for masticatory function training according to the present invention.

### Description of Embodiments

An embodiment (example) of the present invention will be described in more detail below with reference to the drawings.

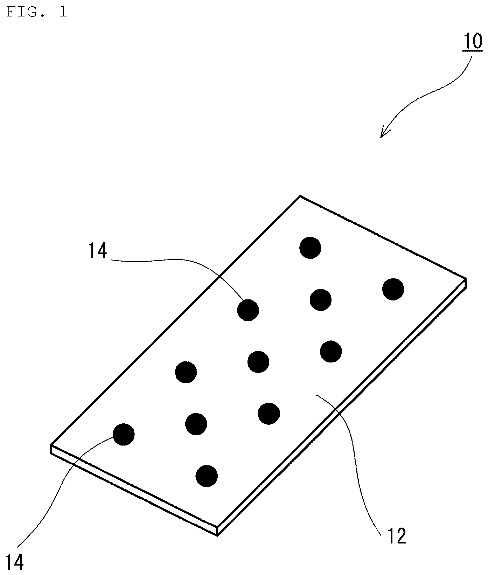

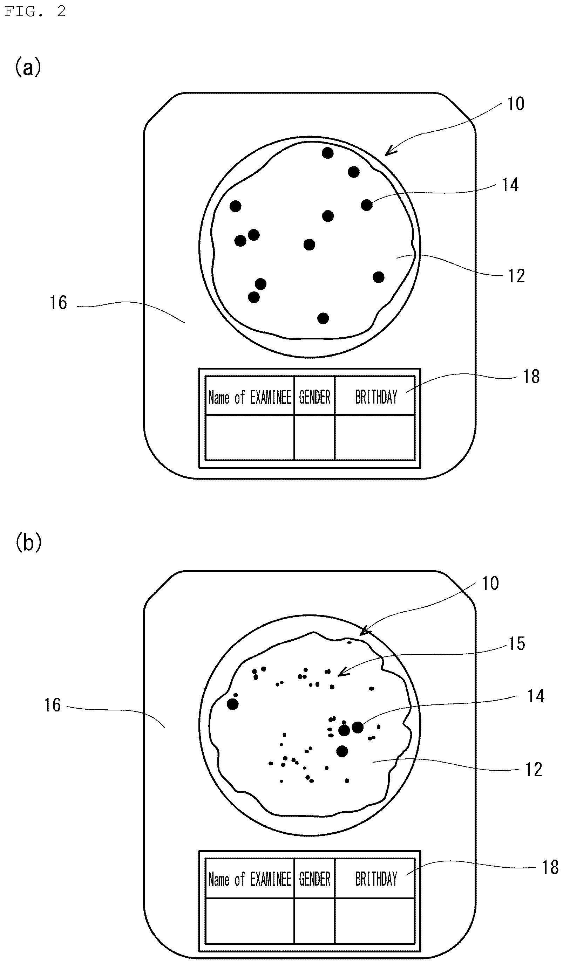

Fig. 1 is a schematic view referenced to explain an example of an artificial food mass for masticatory function evaluation according to the present invention, and Fig. 2 is a schematic view referenced to explain a state of the artificial food mass for masticatory function evaluation, illustrated in Fig. 1, after stretching the artificial food mass. More specifically, Fig. 2(a) is a schematic view illustrating the stretched state of the artificial food mass for masticatory function evaluation, the artificial food mass being not yet masticated, and Fig. 2(b) is a schematic view illustrating the stretched state of the artificial food mass for masticatory function evaluation after a person has masticated the artificial food mass a predetermined number of times.

An artificial food mass 10 for masticatory function evaluation (hereinafter also simply called an "artificial food mass 10") according to this example is constituted by a base material 12 and fine particles 14 contained in the base material 12. The fine particles 14 have properties that the particles are crushed and ground finely by mastication, and have a spherical shape when not masticated.

The base material 12 is not limited to a particular type of material insofar as the material is not harmful to a human body when it is put into the mouth. For example, a natural resin such as chicle, or a synthetic resin such as polyvinyl acetate or polyisobutylene may be used as the base material 12. Preferably, the base material 12 may have a similar composition to that of a commercialized chewing gum, for example.

The fine particles 14 are not limited to a particular type of particles insofar as the particles have such properties as being crushed and ground finely by mastication and have a spherical shape when not masticated, and insofar as the particles are not harmful to a human body when they are put into the mouth. The fine particles 14 may be given as, for example, fine particles obtained by shaping any of waxes including natural wax, such as Carnauba wax or beeswax, and synthetic wax into the particulate form, sugar such as white granulated sugar or yellow granulated sugar, edible capsules covered with coatings of gelatin, agar, or starch (e.g., "Seamless Capsules" made by Morishita Jintan Co., Ltd.), or plant seeds such as rice grains, poppy seeds, or Amaranthus seeds. In particular, the edible capsules are preferably used. In the case of using the edible capsules, size adjustment, etc. of the fine particles 14 is easy.

In the case of using the edible capsules as the fine particles 14, because the coatings formed using gelatin, agar, starch, or the like is easily affected by saliva during the mastication, the coatings are preferably treated to be insoluble. For instance, when gelatin is used to form the coatings, the insoluble treatment can be performed by bridging the gelatin.

Outer diameters of the fine particles 14 are preferably set to about 500 µm to about 2 mm. If the outer diameters of the fine particles 14 are smaller than about 500 µm, it would be difficult to visually count the fine particles, or to count the fine particles with an image analysis by referring to photographic data taken by a smartphone, for example. On the other hand, if the outer diameters of the fine particles 14 are greater than about 2 mm, the masticatory efficiency could not be satisfactorily grasped, and the size of the artificial food mass 10 containing a sufficient number of the fine particles 14 would be too large to masticate. In particular, the outer diameters of the fine particles 14 when used in the artificial food mass 10 for masticatory function evaluation are preferably set to about 600 µm to about 1 mm.

The number of the fine particles 14 contained in the artificial food mass 10 is preferably 10 to 500 per 1 g of the artificial food mass 10. In particular, in the case of visually counting the fine particles 14, the number of the fine particles 14 is preferably not more than 100. If the number of the fine particles 14 is less than 10, the masticatory efficiency could not be accurately grasped. If the number of the fine particles 14 is more than 500, it would be difficult to quickly count the fine particles 14. In particular, if the number of the fine particles 14 is more than 100, it would be difficult to quickly count the fine particles 14 in a visual way, which remain in the artificial food mass and still have the spherical shape. The weight of the artificial food mass 10 is not limited to a particular value, but it is preferably set to about 1 g to 1.5 g as with a general chewing gum.

Preferably, the fine particles 14 have a different color from that of the base material 12. By making the colors of the base material 12 and the fine particles 14 different from each other, the fine particles 14 can be easily specified visually or with an image analysis, and the fine particles 14 remaining in the artificial food mass and still having the spherical shape can be easily counted visually or with an image analysis.

While the artificial food mass 10 is just required to contain the predetermined number of the fine particles 14 in the base material 12, the fine particles 14 may be arranged at predetermined positions in the base material 12 shaped into the form of plate, by way of example, as illustrated in Fig. 1.

By arranging the fine particles 14 at the predetermined positions in the base material 12 as described above, an error of the inspection result can be suppressed which may occur due to individual differences among the many artificial food masses 10, and the masticatory efficiency can be measured with higher accuracy.

While the artificial food mass 10 illustrated in Fig. 1 is in the form of a plate, the form of the artificial food mass 10 is not limited to particular one, and the artificial food mass 10 may have a granular or spherical shape. Preferably, the artificial food mass 10 has such a shape as allowing the artificial food mass to be easily masticated by the examinee.

By using the artificial food mass 10 constituted as described above, the human masticatory function can be quickly and easily evaluated as follows.

First, the examinee is urged to masticate the artificial food mass 10 a predetermined number of times. Then, the masticated artificial food mass 10 is stretched using stretching means 16 with optical transparency, such as a laminate film or a prepared slide, for example, and is brought into the state illustrated in Fig. 2(b).

In that state, the fine particles 14 remaining in the artificial food mass 10 and still having the spherical shape (hereinafter also called "spherical fine particles 14") are counted. In this embodiment, the number of the spherical fine particles 14, or a value depending on the number of the spherical fine particles 14 (e.g., a breakage rate of the fine particles represented by a ratio of the number of the fine particles 14 having been crushed and ground finely to the total number of the fine particles 14 contained in the artificial food mass 10) can be used as a value adapted for determining the masticatory performance in terms of masticatory efficiency η.

Through a process of just stretching the artificial food mass 10 illustrated in Fig. 1 into a sheet after once reshaping it into a ball, the artificial food mass 10 not masticated by the person comes into a state in which all the fine particles 14 have the spherical shape as illustrated in Fig. 2(a), whereas the artificial food mass 10 having been masticated by the person comes into a state in which part of the fine particles 14 is crushed and ground finely by the mastication as illustrated in Fig. 2(b).

The spherical fine particles 14 remaining in the artificial food mass 10 may be counted by a method of directly counting the spherical fine particles 14 contained in the stretched artificial food mass 10. In another example, the stretched artificial food mass 10 may be sandwiched between carbon sheets to transfer the spherical fine particles 14 onto the carbon sheets, and the number of the transferred fine particles 14 may be counted.

Because fine particles 15 having been crushed and ground finely by the mastication (hereinafter also called "crushed fine particles 15") are apparently smaller than the spherical fine particles 14, the crushed fine particles 15 are hardly transferred onto the carbon sheets. Even when the crushed fine particles 15 are transferred onto the carbon sheets, the spherical fine particles 14 and the crushed fine particles 15 are easily distinguishable.

Thus, by using the artificial food mass 10 for masticatory function evaluation according to the present invention, the masticatory function can be quickly and easily evaluated, for example, by individual persons or in the field of dental care. Therefore, the individual persons can easily inspect the masticatory efficiency on a periodic basis, while dentists can easily give education and enlightenment regarding the mastication to patients in the field of dental care.

Alternatively, the fine particles 14 remaining in the artificial food mass and still having the spherical shape may be counted using a masticatory-function evaluation and management system 20 constituted as follows.

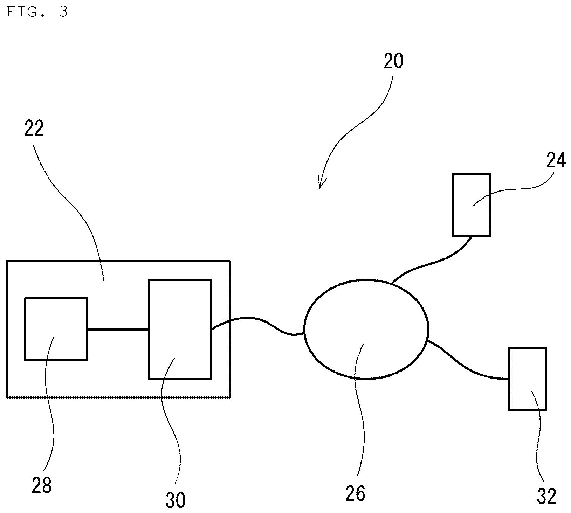

Fig. 3 is a block diagram referenced to explain an example of a masticatory-function evaluation and management system according to the present invention.

The masticatory-function evaluation and management system 20 according to this example includes a server computer 22, as an analyzer, which incorporates a counting program described later, and a network 26 connecting a user terminal 24 and the server computer 22 to each other.

An existing computer can be used as the server computer 22. For example, cloud computing may also be used.

The user terminal 24 is not limited to particular one insofar as it is able to send image data, described later, to the server computer 22. For example, a personal computer (including a desktop PC, a laptop, a tablet, etc.), a smartphone, or a digital camera with a function connectable to the network 26 can be used as the user terminal 24.

The network 26 is not limited to particular one insofar as it is able to connect the server computer 22 and the user terminal 24. The network 26 may be the Internet or an intranet.

The masticatory-function evaluation and management system 20 according to this example further includes a masticatory-function evaluation and management database 28, and database management means 30 for inputting and outputting various data to and from the masticatory-function evaluation and management database 28.

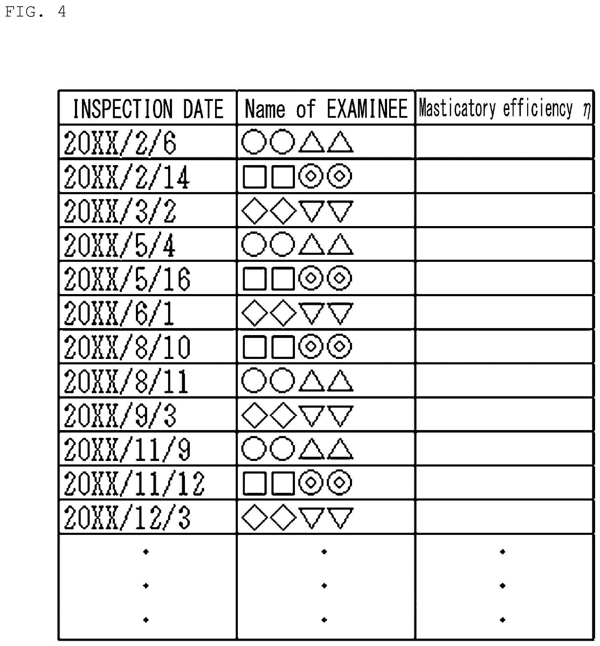

In the masticatory-function evaluation and management database 28, as illustrated in Fig. 4, the masticatory efficiency η quantified using the artificial food mass 10 for masticatory function evaluation is registered in link with examinee information such as the name, gender, age, and birthday of the examinee, and the inspection date.

A counting program operating as described below is installed in the server computer 22 that is an analyzer in the masticatory-function evaluation and management system 20 having the above configuration.

First, the examinee takes an image of the artificial food mass 10 brought into, as described above, the state stretched using the prepared slides, for example, by image-taking means, such as the user terminal 24 with an image-taking function or a digital camera, thus obtaining image data. The image data is not limited to a particular type insofar as the taken image can be handled using the user terminal 24, the server computer 22, etc. For example, a bitmap image may be used for the image data, and any suitable file format may be selected.

The obtained image data of the artificial food mass 10 is sent from the user terminal 24 to the server computer 22 via the network 26.

In the server computer 22, the spherical fine particles 14 present in the image data are counted by a suitable method, such as pattern matching, with reference to the received image data. The pattern matching can be performed by a method disclosed in International Publication No. WO2012/060353, for example.

In the server computer 22, the masticatory efficiency η is calculated on the basis of the number of the spherical fine particles 14 counted as described above. The calculated masticatory efficiency η is input to the masticatory-function evaluation and management database 28 and sent to the user terminal 24 via the network 26 by the database management means 30.

With the configuration described above, the examinee can promptly recognize the masticatory efficiency by taking the image of the artificial food mass 10, which is in the state stretched using stretching means 16, by himself or herself, and by sending the image data to the server computer 22 from the user terminal 24. Thus, there is no need of laborious work such as visually counting the spherical fine particles 14.

When the examinee takes the image by himself or herself, a target 18 for focus adjustment is preferably provided on the stretching means 16 as illustrated in Fig. 2. Because the outer diameters of the fine particles 14 contained in the artificial food mass 10 are as small as about 500 µm to about 2 mm, it is difficult sometimes to adjust focusing on the fine particles 14 when the performance of the image-taking means is not sufficient as in the case of using an inexpensive smartphone, for example. Even in that case, the image of the fine particles 14 having substantially the same focal length as the target 18 can be clearly taken by focusing the image-taking means on the target 18 for focus adjustment, which is provided on the stretching means 16.

The target 18 for focus adjustment is not limited to particular one insofar as the image-taking means is able to adjust focusing on the target 18. Any suitable text or mark may be used as the target 18. In an example, the target 18 may be a text indicating the examinee information such as the name, gender, age, and birthday of the examinee, and the inspection date. In another example, the target 18 may be an identifier, such as a barcode or a QR Code (Registered Trademark), which is linked with the examinee information or which includes the examinee information. The target 18 for focus adjustment may be directly formed on the stretching means 16 by printing, for example, or

By thus providing, as the target 18 for focus adjustment, the text or the identifier containing the examinee information, the examinee information is included in the image data that is sent to the server computer 22 from the user terminal 24 via the network 26.

Thus, in the case of the server computer 22 being equipped with OCR (Optical Character Recognition) means or OMR (Optical Mark Recognition) means, for example, the examinee information can be obtained from the image data, and the masticatory efficiency η and the examinee information can be registered in the masticatory-function evaluation and management database 28 in link with each other by the database management means 30.

The examinee information may be input to the masticatory-function evaluation and management database 28 from the user terminal 24 or a database management terminal 32 by connecting the terminal 24 or 32 to the server computer 22 via the network 26 instead of obtaining the examinee information from the image data.

Furthermore, using the database management terminal 32 makes it possible to perform database management, such as new examinee registration, correction of various data, and optimization of the database, on the masticatory-function evaluation and management database 28.

When the user terminal 24 is connected to the server computer 22 via the network 26, the system is preferably constituted such that various data only related to the examinee utilizing the user terminal 24 can be input to or output from the user terminal 24.

The above-described counting program may be installed into the user terminal 24. In such a case, the user terminal 24 serves as the analyzer.

Thus, since the spherical fine particles 14 present in the image data can be counted at the user terminal 24 using the image data stored in the user terminal 24, the counting of the spherical fine particles 14 and the calculation of the masticatory efficiency η can be performed even when the user terminal 24 is not connected to the network 26.

Also in the above case, the masticatory efficiency η calculated in the user terminal 24, etc. can be registered into the masticatory-function evaluation and management database 28 by sending the masticatory efficiency η, etc. to the server computer 22 when the user terminal 24 is connected to the network 26.

The operations of "inputting and outputting data" in this Description includes, for example, operations of registering (inputting) the masticatory efficiency η, which is linked with the examinee and the inspection date, into the masticatory-function evaluation and management database 28, and displaying (outputting) the masticatory efficiency η having been registered until that time on (to) display means such as a display, which is disposed at the user terminal 24, for example, in link with the inspection date.

According to the masticatory-function evaluation and management system 20 constituted as described above, the examinee, i.e., an individual person, can periodically evaluate the masticatory efficiency η with the aid of the artificial food mass 10 for masticatory function evaluation according to the present invention, and can manage his or her own masticatory efficiency η along the time series by registering the masticatory efficiency η into the masticatory-function evaluation and management database 28.

Therefore, changes of the masticatory efficiency η can be clearly grasped by the individual person, and the enlightening effect of increasing consciousness of the mastication can be given to the examinee. As another effect, the examinee may be prompted to receive diagnosis by a dentist, for example, when the masticatory efficiency η is reduced.

By monitoring the masticatory efficiency η for each examinee by the database management means 30, a message of prompting the examinee to receive diagnosis by a dentist may be displayed at the user terminal 24, for example, when the masticatory efficiency η registered from the user terminal 24 is reduced beyond a predetermined proportion in comparison with the masticatory efficiency η registered before a predetermined period.

While, in the above-described example, the examinee, the inspection date, and the masticatory efficiency η are registered in the masticatory-function evaluation and management database 28, the present invention is not limited to that case. In another example, personal information such as the age and the gender of the examinee, clinical information such as the clinical history and the treatment history of the examinee, etc. may also be registered, and such information may be managed using an individual table prepared in the masticatory-function evaluation and management database 28.

Identification of the examinee utilizing the user terminal 24, which is connected to the database management means 30 via the network 26, can be performed by suitable one of known identification methods. For example, identification using combination of a user ID and a password, biometric identification using a fingerprint or a vocal signature, or identification using an electronic certificate, an IC card or the like may be used.

The masticatory-function evaluation and management database 28 may contain data of primary dentists in link with individual examinees such that the primary dentist of each examinee can input and output various data related to the relevant examinee.

With the configuration described above, when it is authenticated that the user terminal 24 connected to the database management means 30 is utilized by the primary dentist, the primary dentist is able to view various data related to the examinee receiving diagnosis by the primary dentist, and to input the clinical information of the relevant examinee.

Evaluation results of the masticatory function accurately evaluated using the masticatory function evaluation systems, disclosed in PTLs 1 and 2, can be further registered in the masticatory-function evaluation and management database 28.

With the configuration described above, the examinee can more accurately grasp changes in his or her own masticatory efficiency η, and the registered evaluation results can be helpfully used in diagnosis by the main dentist for the examinee.

When reduction of the masticatory function is determined as a result of evaluating the masticatory function using the artificial food mass 10, training of mastication can be practiced with the aid of an artificial food mass 40 for masticatory function training, which is described below.

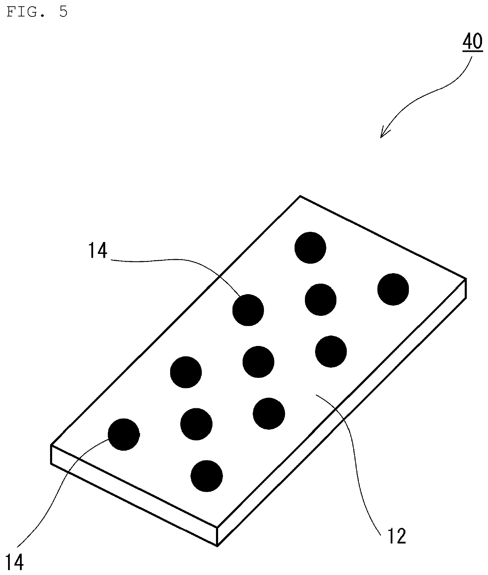

Fig. 5 is a schematic view referenced to explain an example of an artificial food mass for masticatory function training according to the present invention.

The artificial food mass 40 for masticatory function training may basically have substantially the same structure as that of the above-described artificial food mass 10 for masticatory function evaluation. In the following, the same elements are denoted by the same reference signs, and detailed description of those elements is omitted.

In the artificial food mass 40 for masticatory function training, it is important that a user can perceive a feeling of crushing the fine particles 14 upon chewing in an exercise of mastication. Thus, by enabling the user to perceive the feeling of crushing the fine particles 14 upon the chewing, it is possible to urge the user to practice "conscious mastication".

In order that the user can perceive the feeling of crushing the fine particles upon chewing in the exercise of mastication, the following conditions need to be satisfied; namely the outer diameters of the fine particles 14 are set to about 500 µm or more, and the compression strength of the fine particles 14 is set to be greater than that of the base material 12. In particular, the outer diameters of the fine particles 14 are preferably set to about 1 to 1.4 mm.

The fine particles 14 satisfying the above conditions can be surely crushed by the upper and lower teeth with chewing, and the feeling of crushing the fine particles 14 by the teeth can be surely given to the user due to the difference in compression strength between the base material 12 and the fine particles 14.

In this Description, the compression strength of the fine particles 14 stands for maximum stress when the fine particles 14 are forced to break with application of a compression load.

If the compression strength 14 of the fine particles is too great, this would raise a difficulty in crushing the fine particles by the upper and lower teeth upon the chewing, and would damage not only the teeth, but also the periodontal tissues such as the periodontal membrane. For that reason, the compression strength of the fine particles 14 is desirably set to 4.0 kgw/mm² or less.

Preferably, the compression strength of the fine particles 14 and the compression strength of the base material 12 are changed as appropriate depending on the age of the user, symptoms of the periodontal tissues, etc.

When edible capsules are used as the fine particles 14 in the artificial food mass 40 for masticatory function training, an edible dye may be contained in coatings of the edible capsules. With the edible dye contained in the edible capsules as described above, the base material 12 is colored by the edible dye when the fine particles 14 are crushed and ground finely with the mastication.

Thus, since the color of the artificial food mass 40 for masticatory function training is changed with the mastication, whether the mastication is properly performed, i.e., whether the fine particles 14 are properly crushed upon the chewing, can be recognized at a glance depending on the color of the artificial food mass 40 for masticatory function training.

The edible dye is not limited to particular one insofar as it is able to make the base material 12 colored. The color of the edible dye is not limited to particular one, a color being easy to recognize a contrast in shade, such as red, blue, or black, is preferably used. In particular, black is preferable.

Although the preferred embodiment of the present invention has been described above, the present invention is not limited to it, and the present invention can be variously modified within a scope not departing from the object of the present invention.

### Reference Signs List

10 artificial food mass for masticatory function evaluation
12 base material
14 spherical fine particle
15 crushed fine particle
16 stretching means
18 target for focus adjustment
20 masticatory-function evaluation and management system
22 server computer
24 user terminal
26 network
28 masticatory-function evaluation and management database
30 database management means
32 database management terminal
40 artificial food mass for masticatory function training

## Claims

1. An artificial food mass for masticatory function evaluation used to evaluate human masticatory function,
the artificial food mass comprising a base material and fine particles contained in the base material,
wherein the fine particles have a spherical shape when not chewed to be crushed and ground finely with mastication, and
outer diameters of the fine particles are 500 µm to 2 mm.

2. The artificial food mass for masticatory function evaluation according to Claim 1, wherein a number 10 to 500 of the fine particles are contained per 1 g of the artificial food mass for masticatory function evaluation.

3. The artificial food mass for masticatory function evaluation according to Claim 1 or 2, wherein the fine particles are formed of edible capsules, wax, sugar, or plant seeds.

4. A masticatory-function evaluation and management system including an analyzer and performing evaluation and management of human masticatory function,
wherein the analyzer counts the number of fine particles having a spherical shape from among the fine particles present in image data of the artificial food mass, in a stretched state, for masticatory function evaluation according to any one of Claims 1 to 3, and calculates masticatory efficiency on the basis of the number of the fine particles having the spherical shape.

5. The masticatory-function evaluation and management system according to Claim 4, wherein the analyzer is a user terminal.

6. The masticatory-function evaluation and management system according to Claim 5, further including a server computer connected to the user terminal via a network,
wherein the server computer includes a masticatory-function evaluation and management database and database management means to input and output data into and from the masticatory-function evaluation and management database, and
the masticatory-function evaluation and management database is constituted such that the masticatory efficiency is registerable in link with examinee information.

7. A masticatory-function evaluation and management system wherein the analyzer is a server computer connected to a user terminal via a network, and
upon receiving image data of the artificial food mass, in a stretched state, for masticatory function evaluation according to any one of Claims 1 to 3, the image data being sent from the user terminal, the server computer counts the number of fine particles having a spherical shape from among the fine particles present in the image data, and calculates masticatory efficiency on the basis of the number of the fine particles having the spherical shape.

8. The masticatory-function evaluation and management system according to Claim 7, wherein the server computer includes a masticatory-function evaluation and management database and database management means to input and output data into and from the masticatory-function evaluation and management database, and
the masticatory-function evaluation and management database is constituted such that the masticatory efficiency is registerable in link with examinee information.

9. An artificial food mass for masticatory function training used to practice training of mastication,
the artificial food mass comprising a base material and fine particles contained in the base material,
wherein outer diameters of the fine particles are 500 µm or more, and
compression strength of the fine particles is greater than compression strength of the base material.

10. The artificial food mass for masticatory function training according to Claim 7, wherein the compression strength of the fine particles is 4.0 kgw/mm² or less.

11. The artificial food mass for masticatory function training according to Claim 9 or 10, wherein the fine particles are formed of edible capsules, wax, sugar, or plant seeds.
